(19) Europäisches Patentamt / European Patent Office / Office européen des brevets

(11) EP 4 785 980 A1

(12) **DEMANDE DE BREVET EUROPEEN**

(43) Date de publication:
05.08.2026 Bulletin 2026/32

(21) Numéro de dépôt: 25222369.8

(22) Date de dépôt: 10.12.2025

(51) Classification Internationale des Brevets (IPC):
*A61M 16/20* (2006.01)     *A61M 16/00* (2006.01)

(52) Classification Coopérative des Brevets (CPC):
**A61M 16/205; A61M 16/202;** A61M 16/0069;
A61M 16/024; A61M 16/206; A61M 2205/505;
A61M 2205/8212

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: 30.01.2025 FR 2500983

(71) Demandeur: **Air Liquide Medical Systems**
**92182 Antony Cedex (FR)**

(72) Inventeur: BECHIKHI, Taraq
**92182 Antony Cedex (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(54) **VENTILATEUR MÉDICAL À VALVE DE PEP PILOTÉE**

(57) L'invention concerne un ventilateur médical (100) comprenant une turbine motorisée (41) pour fournir un gaz, pilotée par des moyens de pilotage (40). Un circuit de gaz (102) à branche inspiratoire (102.1) et branche expiratoire (102.2) en communication fluidique avec l'atmosphère. Une valve de PEP (1), agencée sur la branche expiratoire (102.2), comprend un clapet (2) coopérant avec un siège de clapet (3). Un dispositif de commande électromécanique (20), piloté par les moyens de pilotage (40), agit sur la face arrière (2.2) du clapet (2) pour le repousser en direction du siège de clapet (3). Une chambre de pressurisation (22) en communication fluidique avec une ligne d'amenée de pression (42) est alimentée par la turbine (41), et coopère avec le clapet (2).

Fig. 2

EP 4 785 980 A1

**Description**

L'invention concerne un appareil de ventilation assistée ou ventilateur médical à valve de PEP pilotée pneumatiquement et électromécaniquement.

Certains respirateurs artificiels, aussi appelés ventilateurs médicaux, utilisés pour ventiler des personnes en ayant besoin, c'est-à-dire des patients, avec un gaz respiratoire, tel de l'air ou un mélange air/oxygène, selon des cycles respiratoires successifs, comprennent une valve expiratoire agencée dans leur circuit pneumatique, par exemple sur une branche expiratoire du circuit pneumatique, c'est-à-dire le circuit de gaz interne de l'appareil. Chaque cycle respiratoire comprend :

- une phase inspiratoire, durant laquelle la valve expiratoire est fermée complétement pour permettre au gaz provenant du ventilateur médical, de pénétrer dans les poumons du patient.
- une phase expiratoire, durant laquelle la valve expiratoire s'ouvre pour permettre au patient d'expirer vers l'extérieur du circuit pneumatique, soit à la pression atmosphérique, soit à une pression supérieure à la pression atmosphérique.

La valve expiratoire sert à opérer des expirations de gaz par le patient à une pression au-dessus de la pression atmosphérique, appelée Pression Expiratoire Positive (PEP) ou pression de PEP. Pour ce faire, la valve expiratoire ou valve de PEP doit être commandée afin qu'elle puisse être soit complétement fermée (en phase inspiratoire), soit au moins partiellement ouverte (en phase expiratoire) pour vidanger le circuit pneumatique et par ailleurs maintenir une pression de PEP dans le circuit pneumatique.

Actuellement, les valves de PEP peuvent être commandées de manière uniquement pneumatique. Ainsi, en phase inspiratoire, une pression gazeuse de commande égale à la pression dans le circuit pneumatique est appliquée à la valve de PEP pour permettre sa fermeture complète, alors qu'en phase expiratoire, une pression gazeuse inférieure à la pression inspiratoire, calculée et régulée par les moyens de pilotage du respirateur, par exemple une carte électronique à microprocesseur, est appliquée à la valve de PEP afin de permettre son ouverture pour vidanger et dépressuriser le circuit pneumatique et maintenir dans celui-ci à la pression de PEP souhaitée.

Les respirateurs, i.e. appareils respiratoires, sont équipés d'une source principale de pression ou de débit pour pressuriser le circuit pneumatique et appliquer au patient la pression souhaitée. La source principale de pression ou de débit peut être par exemple une turbine motorisée, aussi appelée compresseur, (micro)soufflante ou analogue, commandée par les moyens de pilotage du respirateur. Les termes turbine, compresseur et (micro)soufflante sont considérés comme équivalents et substituables.

La turbine motorisée peut servir de source de pression servant à commander la valve expiratoire, i.e. la valve de PEP. Dans ce cas :

- en phase inspiratoire, le moteur électrique de la turbine est commandé pour accélérer, fournir le gaz respiratoire, et ainsi pressuriser le circuit pneumatique et délivrer au patient la pression ou le débit inspiratoire souhaité. La pression de commande de la valve de PEP est alors égale à celle du gaz en sortie turbine et donc à celle qui règne dans le circuit pneumatique du patient, ce qui permet la fermeture complète de la valve de PEP.
- en phase expiratoire, une pression inférieure à la pression inspiratoire, calculée et régulée par le respirateur, est appliquée à la valve de PEP afin de permettre son ouverture pour vidanger le circuit pneumatique et maintenir dans celui-ci la pression dite de PEP. La turbine décélère, i.e. ralentit, et fournit un très faible débit gazeux, appelé « flow by », pour permettre la régulation de la pression de PEP dans le circuit patient. La commande de pression de PEP provient soit de la pression fournie par la turbine, soit d'une autre source de pression. La commutation de la commande de pression de PEP de la phase inspiratoire vers la phase expiratoire est assurée par des électrovannes.

Toutefois, opérer une commande uniquement pneumatique de la valve de PEP présente des inconvénients et des problèmes, notamment un temps de réponse assez long qui empêche de ventiler les patients à fréquence respiratoire élevée ; une création d'un frein pneumatique, plus ou moins important à l'expiration, en fonction des réglages de la machine ; et lorsque la pression de PEP réglée est élevée, i.e. supérieure à 10 hPa environ, la régulation de la PEP devient plus difficile et la valve de PEP commence à créer des vibrations pneumatiques dans le circuit, plus ou moins audibles, qui provoquent des dysfonctionnements du système de déclenchement du respirateur.

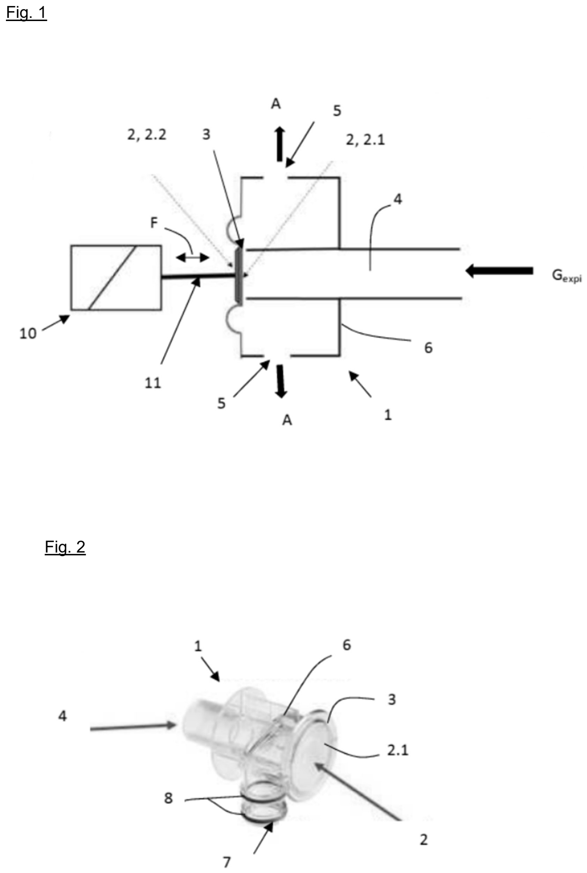

Pour tenter d'y remédier, il a été proposé d'utiliser une commande électromécanique mettant en oeuvre un actionneur électromécanique proportionnel, appelé VCA (*Voice Coil Actuator* ou actionneur à bobine mobile), qui vient agir mécaniquement sur le clapet de la valve de PEP pour le décoller ou non de son siège et ainsi ouvrir ou fermer la valve de PEP, comme illustré en Fig. 1.

Si cette solution évite les problèmes susmentionnés, elle présente un inconvénient majeur, à savoir d'engendrer une consommation de courant qui est bien plus importante qu'un système à commande pneumatique, ce qui n'est pas acceptable.

Un problème est dès lors de pouvoir proposer un ventilateur à valve de PEP pilotée par un système de commande amélioré permettant de conserver les avantages d'un système de commande électromécanique tout en réduisant notablement la consommation électrique qui en découle, et par ailleurs sans rencontrer les problèmes et inconvénients d'un système de commande uniquement pneumatique.

Une solution selon l'invention concerne un ventilateur médical, i.e. un respirateur, comprenant une turbine motorisée pour fournir un gaz ; des moyens de pilotage pour piloter la turbine ; un circuit de gaz comprenant une branche inspiratoire pour véhiculer le gaz provenant de la turbine et une branche expiratoire en communication fluidique avec l'atmosphère ; et une valve de PEP, agencée sur la branche expiratoire, comprenant un clapet comprenant une face avant coopérant avec un siège de clapet, et une face arrière opposée à la face avant.

De plus, le ventilateur médical comprend en outre un dispositif de commande électromécanique, piloté par les moyens de pilotage, agissant sur la face arrière du clapet pour le repousser en direction du siège de clapet, et une chambre de pressurisation en communication fluidique avec une ligne d'amenée de pression alimentée par la turbine, ladite chambre de pressurisation coopérant avec la face arrière du clapet.

Selon le mode de réalisation considéré, le ventilateur de l'invention peut comprendre l'une ou plusieurs des caractéristiques suivantes :

- le dispositif de commande électromécanique agit sur la face arrière du clapet par l'intermédiaire d'un actionneur.
- la chambre de pressurisation est agencé du côté de la face arrière du clapet et délimitée par au moins une partie de la surface de ladite face arrière du clapet.
- le clapet est en un matériau souple, de préférence en silicone ou tout autre matériau adapté.
- le clapet comprend une chambre interne formant la chambre de pressurisation.
- le dispositif de commande électromécanique comprend une bobine et un aimant mobiles relativement l'un par rapport à l'autre.
- la bobine est commandée par les moyens de pilotage.
- la ligne d'amenée de pression comprend un conduit d'échappement à l'atmosphère, agencé entre la turbine et la valve de PEP.
- le conduit d'échappement à l'atmosphère équipé d'un orifice calibré.
- les moyens de pilotage comprennent au moins un (micro)processeur.
- les moyens de pilotage comprennent au moins une carte électronique à microprocesseur.
- il comprend des moyens d'alimentation électrique fournissant de l'énergie électrique aux moyens de pilotage, à la turbine motorisée et/ou au dispositif de commande électromécanique.
- le circuit de gaz comprend des passages, des conduits ou analogues.

L'invention concerne aussi une méthode de ventilation d'une personne en ayant besoin, i.e. un patient, avec un gaz respiratoire, tel de l'air ou un mélange air/oxygène (i.e. air enrichi en $O_2$), fourni par un ventilateur médical selon l'invention, selon des cycles respiratoires successifs comprenant une phase inspiratoire durant laquelle la valve expiratoire (i.e. la valve de PEP) est commandée ou pilotée pour être complétement fermée pour permettre au gaz provenant du ventilateur médical de pénétrer dans les poumons du patient (donc l'empêcher d'être évacué vers l'extérieur du circuit pneumatique) ; et une phase expiratoire durant laquelle la valve expiratoire (i.e. la valve de PEP) est commandée ou pilotée pour s'ouvrir pour permettre au patient d'expirer vers l'extérieur du circuit pneumatique.

**Définitions**

Dans le cadre de l'invention :

- les termes « conduit », « tuyau », « canalisation » ou « ligne » sont considérés comme équivalents et substituables.
- les termes « appareil de ventilation assistée », « ventilateur médical » et « respirateur artificiel » sont considérés comme équivalents et substituables.
- les termes « turbine », « compresseur » et « soufflante » sont considérés comme équivalents et substituables.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens d'alimentation électrique » peuvent être remplacés par « dispositif d'alimentation électrique »...
- par « mesure de pression », on entend une valeur de pression (e.g. une valeur numérique) ou un signal représentatif d'une telle valeur de pression reflétant ou correspondant à la pression gazeuse mesurée par un capteur de pression ou analogue.
- par « mesure de débit », on entend une valeur de débit (e.g. une valeur numérique) ou un signal représentatif d'une telle valeur de débit reflétant ou correspondant à un débit mesuré par un capteur de débit, tel un capteur de débit massique, ou à des valeurs de pression mesurées par un capteur de pression différentiel ou analogue puis traitées et/ou converties en débit par exemple au sein des moyens de pilotage.

- les termes « amont » et/ou « aval » sont utilisés par rapport au sens normal de circulation du gaz, à savoir dans le sens allant vers le patient.

L'invention va maintenant être mieux comprise grâce à la description détaillée suivante, faite à titre illustratif mais non limitatif, en référence aux figures annexées parmi lesquelles :

- Fig. 1 schématise un système de commande électromécanique par actionneur électromécanique proportionnel selon l'art antérieur apte à agir sur une valve de PEP de ventilateur médical.
- Fig. 2 schématise un mode de réalisation d'une valve de PEP pour ventilateur médical selon l'invention.
- Fig. 3 schématise un premier mode de réalisation selon l'invention d'un système de commande de la valve de PEP de Fig. 2.
- Fig. 4 schématise un deuxième mode de réalisation selon l'invention d'un système de commande de la valve de PEP de Fig. 2.
- Fig. 5 schématise un troisième mode de réalisation selon l'invention d'un système de commande de la valve de PEP de Fig. 2.
- Fig. 6 schématise l'architecture et le fonctionnement d'un ventilateur médical à valve de PEP.

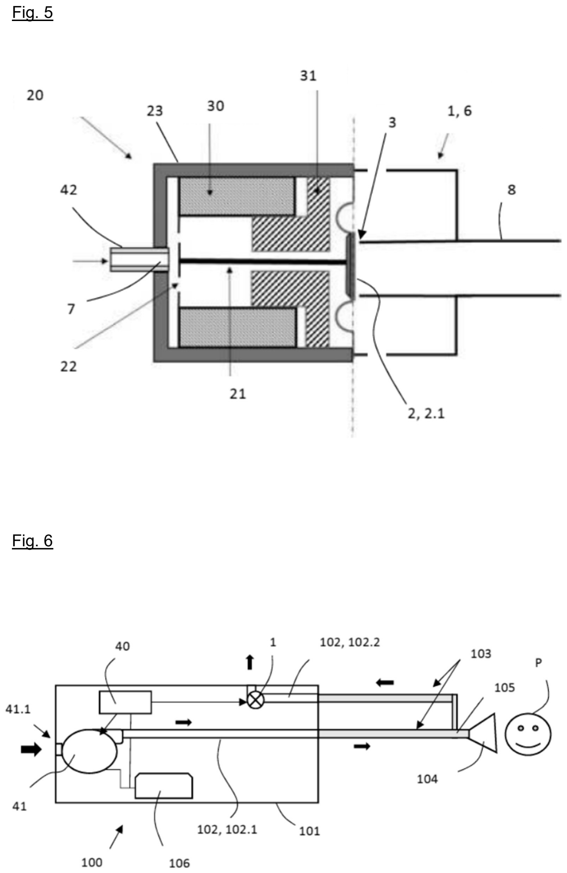

Fig. 6 schématise l'architecture et le fonctionnement d'un appareil de ventilation ou ventilateur médical 100 comprenant une valve de PEP 1. Le ventilateur médical 100 comprend une carcasse ou coque externe 101, par exemple en polymère, dans laquelle sont agencés les différents composants de l'appareil 100, notamment la turbine motorisé 41, i.e. (micro-)soufflante, compresseur ou analogue, aspirant de l'air ambiant via une entrée d'air 41.1 et par ailleurs reliée fluidiquement, en sortie, au circuit de gaz interne 102 du ventilateur 1, qui comprend ici une branche inspiratoire 102.1 et une branche expiratoire 102.2 équipée d'une valve de PEP 1, typiquement des conduits ou passages de gaz.

La turbine motorisé 41 fournit le gaz sous pression à la branche inspiratoire 102.1 du circuit de gaz interne 102 qui le véhicule en direction du patient P. Les branches inspiratoire 102.1 et expiratoire 102.2 sont raccordées à un circuit patient externe 103, tels des tuyaux flexibles se raccordant au niveau d'une pièce en Y 105, permettant d'acheminer le gaz, e.g/ air, provenant de la branche inspiratoire 102.1 jusqu'à une interface respiratoire 104, tel un masque respiratoire, servant à fournir le gaz au patient P et, par ailleurs, à évacuer le gaz expiré ($G_{expi}$) par le patient P vers la branche expiratoire 102.2 qui permet d'évacuer le gaz expiré riche en $CO_2$ vers l'atmosphère.

Typiquement, le gaz respiratoire fourni par la turbine 41 est de l'air ou de l'air enrichi en oxygène, voire de l'oxygène pur, notamment en fonction de la thérapie/ventilation à mettre en oeuvre. L'oxygène peut être fourni par une source externe, par exemple une prise murale alimentée par une canalisation de gaz ou par une bouteille d'oxygène venant alimenter le ventilateur 100 en oxygène additionnel, par exemple en amont de la turbine 41 (non montré).

La turbine 41 est motorisée, c'est-à-dire qu'elle comporte un moteur électrique, typiquement un moteur sans balais (i.e. *brushless*) ayant une vitesse de rotation pouvant atteindre 40 000 tr/min à 70 000 tr/min, et préférentiellement ayant aussi une faible inertie. Classiquement, une telle turbine 41 comprend un moteur électrique protégé par un carter et entraînant, pendant son fonctionnement, un axe ou arbre rotatif portant une roue à ailettes agencée dans le compartiment interne d'une volute qui surmonte le moteur électrique et le carter. La volute comprend une entrée de gaz par lequel l'air aspiré pénètre dans le compartiment interne et une sortie de gaz par laquelle le gaz sous pression ressort du compartiment interne puis alimente la branche inspiratoire 101.2 du circuit de gaz interne 102. La volute peut être surmontée d'un pavillon.

Afin de contrôler la circulation de gaz dans le circuit de gaz interne 102, en particulier dans la branche inspiratoire 101.2 en direction du patient P, on peut prévoir différents éléments additionnels, comme un ou des électrovanne(s), un ou des capteurs de pression et/ou de débit ou autres.

Sont également prévus des moyens de pilotage 40 qui reçoivent et traitent les mesures de pression et/ou débit opérés par les capteurs, et par ailleurs pilotent la ou les électrovanne(s) et/ou les accélérations et décélérations du moteur de la turbine 41 en fonction des phases respiratoires du patient P.

Les moyens de pilotage 40 comprennent avantageusement un (ou des) microprocesseur mettant en oeuvre un ou plusieurs algorithmes, de préférence un (ou des) microcontrôleur. Typiquement, le (les) microprocesseur est porté par une (ou des) carte électronique.

Plus précisément, la turbine 41 est pilotée par les moyens de pilotage 40, par exemple via une valeur de tension continue générée à l'aide d'un signal tout-ou-rien, typiquement un pilotage par modulation de largeur d'impulsion (i.e. PWM pour *Pulse-Width Modulation* en anglais), ou via une valeur de courant et est régulée grâce à un monitorage de la pression, notamment via les données de pression retournées par un capteur de pression.

Les moyens de pilotage 40 peuvent aussi comprendre et/ou coopérer avec des moyens de mémorisation, telle une ou des mémoires d'enregistrement des données, par exemple une mémoire rapide, i.e. mémoire flash, faisant office de mémoire morte et par ailleurs une mémoire vive ou RAM (i.e. *random* access *memory*).

La mémoire flash permet d'enregistrer et conserver le paramétrage de modes ventilatoires, c'est-à-dire les valeurs de pression, débit etc... associées à chaque mode ventilatoire, ou d'autres données, informations, paramètres de fonctionnement ou autres... devant être conservés au sein de l'appareil 100. Les modes ventilatoires mémorisées sont par exemple un mode CPV, un mode CPAP, VPAC, VSAI, HFOT ou tout autre mode de ventilation.

Par ailleurs, la mémoire RAM permet de mémoriser temporairement des modifications éventuelles de paramètres souhaitées par l'utilisateur ou toute autre donnée, information ou paramètre provisoire. Ces modifications peuvent être entrées dans le ventilateur 100 au moyen, par exemple, d'une interface graphique utilisateur (IGU) ou une interface homme-machine (IHM) comprenant des moyens de réglage (e.g., boutons, touches, curseurs ou autres) et un écran d'affichage graphique en couleurs ou en noir et blanc. L'écran d'affichage graphique peut être un écran tactile et les moyens de réglage de l'IGU ou de l'IHM servant modifier ou ajuster des réglages ventilatoires (i.e. paramètres) sont alors préférentiellement des touches virtuelles ou analogues s'affichant sur ledit écran tactile.

Des moyens d'alimentation électrique 106 alimentent les composants du ventilateur 100 ayant besoin de courant électrique pour fonction, par exemple la turbine 41, les moyens de pilotage 40, l'IHM, les capteurs... Les moyens d'alimentation électrique 106 peuvent comprendre une alimentation secteur 110/220V (e.g. câble, prise électrique...) et/ou une batterie rechargeable interne

Enfin, il est à souligner que la circulation du gaz dans la branche expiratoire 102.2 est contrôlée par une valve expiratoire, à savoir une valve de PEP 1, agencée sur ladite branche expiratoire 102.2 du ventilateur 100, comme détaillé ci-après.

Le ventilateur 1 est configuré pour fonction selon des cycles respiratoires, où chaque cycle respiratoire comprend :

- une phase inspiratoire, durant laquelle la valve expiratoire 1 est fermée complétement pour permettre au gaz provenant de la turbine 41 du ventilateur médical 100 d'être acheminé jusqu'au patient P par la branche inspiratoire 102.1, le circuit patient 103 et l'interface respiratoire 104, puis de pénétrer dans les poumons du patient P.
- une phase expiratoire, durant laquelle la valve expiratoire 41 s'ouvre pour permettre au patient d'expirer, soit à la pression atmosphérique, soit à une pression supérieure à la pression atmosphérique (i.e. pression de PEP). Le gaz expiré par le patient dans l'interface 104, chemine donc ensuite par le circuit patient 103, la branche expiratoire 102.2 du ventilateur 100 et la valve de PEP 1, avant d'être rejeté à l'atmosphère ambiante.

La valve de PEP 1 doit donc être commandée ou pilotée pour assurer son ouverture ou sa fermeture en fonction des cycles respiratoires successifs.

Ainsi, Fig. 1 schématise un système de commande électromécanique de la valve expiratoire ou valve de PEP 1 d'un ventilateur médical 100, tel celui de Fig. 6, au moyen d'un actionneur électromécanique proportionnel 10, 11 comprenant un axe d'actionnement 11, ou VCA selon l'art antérieur.

L'actionneur 10, 11 vient agir mécaniquement, via l'axe 11, en appliquant une force variable (en F) sur la face arrière 2.2 du clapet 2 de la valve 1 de PEP, auquel il est solidarisé, pour :

- pendant les phases expiratoires, décoller la face avant 2.1 du clapet 2 du siège de clapet 3 et ainsi ouvrir la valve de PEP 1 pour laisser s'échapper le gaz vers l'atmosphère A, via le ou les orifices de sortie 5 aménagés dans le corps de valve 6, typiquement le gaz expiré ($G_{expi}$) riche en $CO_2$ amené par le conduit d'alimentation 4 relié à la branche expiratoire du circuit patient ou
- à l'inverse, pendant les phases inspiratoires, le repousser contre le siège de clapet 3 et ainsi fermer la valve 1 de PEP, de manière à boquer la sortie du gaz vers l'atmosphère A, via le ou les orifices de sortie 5, et permettre un apport de gaz respiratoire au patient.

Or, l'utilisation d'un tel système de commande électromécanique 10 dans un ventilateur 100, tel celui de Fig. 6, présente l'inconvénient d'engendrer une consommation élevée de courant électrique.

Fig. 2 schématise un mode de réalisation d'une valve de PEP 1 selon l'invention destinée à être agencée sur la branche expiratoire 102.2 du circuit de gaz interne, i.e. le circuit pneumatique, d'un ventilateur médical 100, tel celui de Fig. 6 décrit ci-avant.

La valve de PEP 1 selon l'invention comprend un corps de valve 6 comprenant un clapet 2 coopérant avec un siège de clapet 3 pour autoriser ou interdire le passage de gaz au travers du corps de valve 6, en direction d'un orifice de sortie 7 porté par un conduit de sortie 8 en communication fluidique avec l'atmosphère ambiante.

Le corps de valve 6 comprend en outre un conduit d'alimentation 4 pour amener le gaz expiré ($G_{expi}$) par le patient, qui est riche en $CO_2$. Ce conduit d'alimentation 4 est destiné à être raccordé fluidiquement à la branche expiratoire 102.2 du ventilateur 100 qui est alimenté avec le gaz expiré par le patient P.

Dans ce cas, la pression gazeuse issue de la turbine 41 vient s'appliquer sur le clapet 2 pour contrôler l'ouverture/fermeture de la valve de PEP 1.

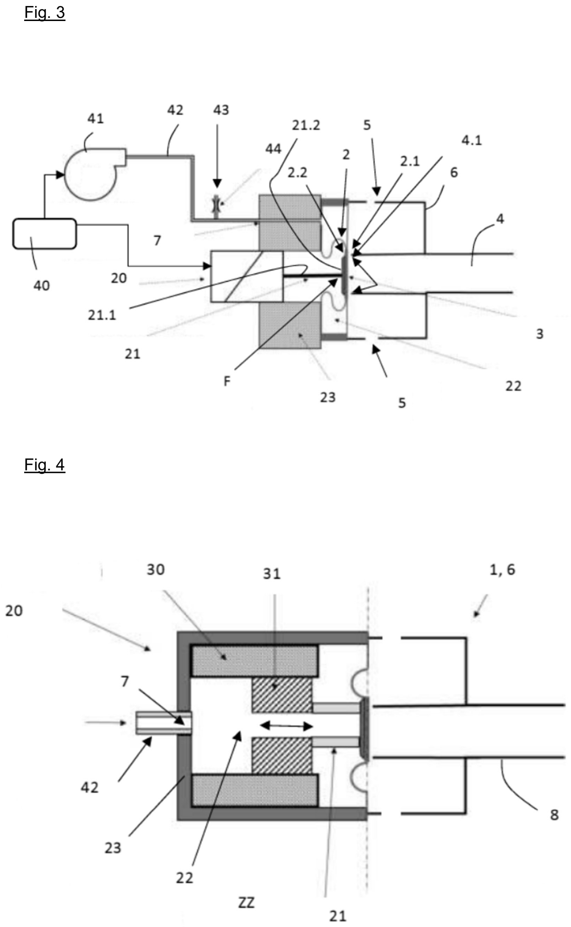

Fig. 3 schématise un premier mode de réalisation d'un système de commande mixte 20, 42 d'une valve de PEP 1 selon l'invention, telle la valve 1 de Fig. 2, d'un ventilateur médical 100, tel celui de Fig. 6 décrit ci-avant.

Le système de commande mixte 20, 42 selon l'invention est basé sur une double commande de la valve de PEP 1 combinant une pression pneumatique amenée par une ligne pneumatique 42 alimentée par le gaz fourni par la turbine 41 et une force électromécanique F agissant sur la face arrière 2.2 du clapet 2 de la valve 41, via système de commande électromécanique comprenant un actionneur 20, 21 avec axe 21.1, afin de fermer ou d'ouvrir la valve 1 d'une manière proportionnelle, en repoussant plus ou moins le clapet 2 en direction du siège de clapet 3.

En d'autres termes, le système de commande mixte 20, 42 associé à la valve de PEP 1 d'un ventilateur 100 selon l'invention comprend :

- un dispositif de commande électromécanique 20, piloté par les moyens de pilotage 40 du ventilateur 100, typiquement un (micro)processeur d'une carte électronique, agissant sur la face arrière 2.2 du clapet 2 pour le repousser en direction du siège de clapet 3, en lui appliquant une force (F) proportionnelle à l'ouverture souhaitée de la valve de PEP 1, pendant les phases expiratoires, et
- une chambre de pressurisation 22 en communication fluidique avec une ligne d'amenée de pression 42 alimentée par la turbine 41 pour recevoir du gaz sous pression, ladite chambre de pressurisation 22 coopérant avec la face arrière 2.2 du clapet 2 de manière à y appliquer une pression pneumatique, i.e. gazeuse, comme expliqué ci-après.

Le dispositif (i.e. système) de commande électromécanique comprend un actionneur 20, 21 venant agir contre la face arrière 2.2 du clapet 2 pour le repousser en direction du siège de clapet 3. L'actionneur 20, 21 peut être solidarisé, i.e. fixé, à ladite face arrière 2.2 du clapet 2.

L'actionneur 20, 21 est mû par un électro-aimant proportionnel (VCA).

Le dispositif (i.e. système) de commande électromécanique, en particulier l'actionneur 20, 21, est au moins partiellement agencé aussi dans un boitier 23 venant se solidariser au corps de valve 6 de PEP. L'actionneur 21 est mobile dans le boitier 23.

Dans le mode de réalisation de Fig. 3, l'actionneur 21 comprend un axe 21.1, telle une tige ou analogue, portant une tête 21.2, ici en forme de disque, solidarisée à la face arrière 2.2 du clapet 2, par exemple par collage ou autre.

Le clapet 2 est préférentiellement en matériau souple, i.e. déformable ou flexible, par exemple en silicone ou tout autre matériau adapté.

La face avant 2.1 du clapet 2 coopère avec le siège de clapet 3 comprenant la bordure terminale 4.1 du conduit 4 pour contrôler le passage de gaz.

Lorsque la face avant 2.1 du clapet 2 vient en appui contre le siège de clapet 3, il s'opère une fermeture étanche du conduit 4 qui empêche toute circulation de gaz. A l'inverse, lorsque la face avant 2.1 du clapet 2 n'est pas ou plus en appui contre le siège de clapet 3, il s'opère une ouverture du conduit 4 permettant ainsi une circulation de gaz vers l'atmosphère extérieure.

Autrement dit, l'axe 21.1 et le clapet 2 peut être poussé ou tiré, selon si l'on souhaite ouvrir ou fermer la valve de PEP 1.

Dans ce premier mode de réalisation, le clapet 2 utilisé a une structure particulière puisqu'il comprend la chambre de pressurisation 22, c'est-à-dire une chambre de pressurisation interne, qui reçoit la pression gazeuse amenée par la ligne d'amenée de pression 42, tel un conduit ou analogue, afin d'assurer un pilotage pneumatique de la valve 1.

La ligne d'amenée de pression 42 comprend un conduit d'échappement à l'atmosphère 43, agencé entre la turbine 41 et la valve 1, i.e. de préférence un conduit ou passage, équipé d'un orifice calibré 44. Le conduit d'échappement à l'atmosphère 43 permet d'accélérer la dépressurisation de la chambre de pressurisation 22 de la valve de PEP 1, lors du passage d'une phase d'inspiration à une phase d'expiration.

La ligne d'amenée de pression 42 peut être reliée fluidiquement à la chambre interne 22 du clapet 2, via un canal 7 ou analogue traversant la paroi du boitier 23.

En fonctionnement, en particulier pendant les phases expiratoires, le dispositif de commande électromécanique applique sur la face arrière 2.2 du clapet 2, via l'actionneur 20, 21, une force F plus ou moins importante, i.e. une force proportionnelle, qui est dirigée vers le siège 3 de manière à fermer plus ou moins fortement la valve de PEP 1, comme expliqué ci-avant, c'est-à-dire que la force F appliquée est proportionnelle à la pression de PEP.

Dès lors, durant la phase expiratoire, la turbine 41 freine et fait diminuer la pression dans la ligne d'amenée de pression 42, grâce à quoi la pression dans la chambre diminue avec la fuite du gaz via le conduit d'échappement à l'atmosphère 43 portant l'orifice de sortie de gaz.

En l'absence de pression s'exerçant dans la chambre de pressurisation 22 du clapet 2, la valve expiratoire, i.e. la valve de PEP 1, peut être fermée par la force F qui est directement proportionnelle à la pression qui règne dans le circuit pneumatique du patient, laquelle est donnée par la relation :

**EP 4 785 980 A1**

Pression = Force F / Surface du siège du clapet.

L'application d'une telle pression sur le clapet 2 nécessite une consommation électrique de la part de du dispositif de commande électromécanique 20 qui est d'autant plus importante que la surface du siège de clapet et/ou que la pression du circuit est importante(s).

Or, la pression gazeuse délivrée par la turbine 41 qui est acheminée par la ligne d'amenée de pression 42 et le canal 7, vient directement s'appliquer dans la chambre de pressurisation 22 du clapet 2 de la valve de PEP 1, c'est-à-dire ici dans sa chambre de pressurisation interne.

Cette pression pneumatique s'exerçant dans la chambre de pressurisation 22 du clapet 2, va aussi s'appliquer sur la face arrière 2.2 du clapet 2, en plus de la force F exercée par l'actionneur 21 du dispositif de commande électromécanique 20, ce qui va permettre une réduction importante de la force F devant être appliquée par le dispositif de commande électromécanique 20 et par conséquent de diminuer aussi la quantité d'énergie consommée par ce dispositif de commande électromécanique 20.

Fig. 4 et Fig. 5 schématisent un deuxième et un troisième mode de réalisation d'un système de commande de la valve de PEP 1 de Fig. 2 selon l'invention, qui fonctionnent selon le principe exposé ci-avant et décrit en relation avec le premier mode de réalisation de Fig. 3 ; les éléments en commun ne sont dès lors pas redétaillés ci-après.

Dans le deuxième mode de réalisation de Fig. 4, le dispositif de commande électromécanique 20 comprend une bobine électrique 30 fixe coopérant avec un aimant 31 mobile portant un actionneur 21, tel une pièce intermédiaire collée à l'aimant, lequel actionneur 21 est, là encore, solidarisé à la face arrière 2.2 du clapet 2.1 de manière à pouvoir exercer une force mécanique proportionnelle sur ledit clapet 2, comme expliqué ci-avant.

La bobine électrique 30 et l'aimant 31 sont agencés dans le boitier 23.

La bobine électrique 30 est commandée par les moyens de pilotage 40 pour opérer les déplacements en translation de l'aimant 31 mobile, notamment en direction du clapet 2. Ceci est le principe de fonctionnement d'un électro-aimant proportionnel à aimant mobile.

Comme déjà dit, la force s'appliquant sur l'actionneur peut être dans les 2 directions, c'est-à-dire permettant de pousser ou de tirer le clapet de la valve de PEP 1.

La bobine électrique 30 consomme du courant électrique durant son fonctionnement comme dans le premier mode de réalisation.

Là encore, on prévoit une chambre de pressurisation 22 alimentée en pression gazeuse par la ligne d'amenée de pression 42 alimentée par la turbine 41, et coopérant avec la face arrière 2.2 du clapet 2 de sorte que la pression qui y règne puisse venir s'exercer sur la face arrière 2.2 du clapet 2, comme déjà expliqué, ce qui permet de réduire, là aussi, la consommation d'énergie électrique de la bobine 30.

Dans ce cas, la chambre de pressurisation 22 est agencée dans le boitier 23 et est alimentée en gaz via un canal 7 traversant la paroi, par exemple la paroi arrière, du boitier 23, lequel canal 7 est alimenté, comme précédemment, par la ligne d'amenée de pression 42 qui vient s'y raccorder.

Fig. 5 schématise un troisième mode de réalisation selon l'invention qui est globalement analogue à celui du deuxième mode de réalisation de Fig. 4 mais fonctionne de manière inverse.

En effet, dans ce troisième mode de réalisation, le dispositif de commande électromécanique 20 comprend aussi une bobine électrique 30 coopérant avec un aimant 31 mais ici, l'aimant 31 est fixe, i.e. immobile, alors que la bobine électrique 30 est mobile en translation et porte l'actionneur 21 qui est solidarisé à la face arrière 2.2 du clapet 2.1 de manière à pouvoir exercer une force mécanique proportionnelle sur ledit clapet 2, comme expliqué ci-avant.

Comme dans le deuxième mode de réalisation, une chambre de pressurisation 22 alimentée en pression gazeuse par la ligne d'amenée de pression 42 alimentée par la turbine 41, est présente du côté de la face arrière 2.2 du clapet 2 de sorte que la pression qui y règne puisse venir s'exercer sur la face arrière 2.2 du clapet 2, comme déjà expliqué, ce qui permet de réduire, là aussi, la consommation d'énergie électrique de la bobine 30.

**Revendications**

1.  Ventilateur médical (100) comprenant :

    - une turbine motorisée (41) pour fournir un gaz,
    - des moyens de pilotage (40) pour piloter la turbine (41),
    - un circuit de gaz (102) comprenant une branche inspiratoire (102.1) pour véhiculer le gaz provenant de la turbine (41) et une branche expiratoire (102.2) en communication fluidique avec l'atmosphère, et
    - une valve de PEP (1), agencée sur la branche expiratoire (102.2), comprenant un clapet (2) comprenant une face avant (2.1) coopérant avec un siège de clapet (3), et une face arrière (2.2) opposée à la face avant (2.1),

**caractérisé en ce qu'**il comprend en outre :

- un dispositif de commande électromécanique (20), piloté par les moyens de pilotage (40), agissant sur la face arrière (2.2) du clapet (2) pour le repousser en direction du siège de clapet (3), et
- une chambre de pressurisation (22) en communication fluidique avec une ligne d'amenée de pression (42) alimentée par la turbine (41), ladite chambre de pressurisation (22) coopérant avec la face arrière (2.2) du clapet (2).

2. Ventilateur selon la revendication 1, **caractérisé en ce que** le dispositif de commande électromécanique (20) agit sur la face arrière (2.2) du clapet (2) par l'intermédiaire d'un actionneur (21).

3. Ventilateur selon la revendication 1, **caractérisé en ce que** la chambre de pressurisation (22) est agencé du côté de la face arrière (2.2) du clapet (2) et délimitée par au moins une partie de la surface de ladite face arrière (2.2) du clapet (2).

4. Ventilateur selon l'une des revendications 1 à 3, **caractérisé en ce que** le clapet (2) est en un matériau souple.

5. Ventilateur selon l'une des revendications 1, 3 ou 4, **caractérisé en ce que** le clapet (2) comprend une chambre interne formant la chambre de pressurisation (22).

6. Ventilateur selon la revendication 1, **caractérisé en ce que** le dispositif de commande électromécanique (20) comprend une bobine (30) et un aimant (31) mobiles relativement l'un par rapport à l'autre.

7. Ventilateur selon les revendications 1 et 6, **caractérisé en ce que** la bobine (30) est commandée par les moyens de pilotage (40).

8. Ventilateur selon la revendication 1, **caractérisé en ce que** la ligne d'amenée de pression (42) comprend un conduit d'échappement à l'atmosphère (43), agencé entre la turbine (41) et la valve de PEP (1).

9. Ventilateur selon la revendication 1, **caractérisé en ce que** les moyens de pilotage (40) comprennent au moins un processeur.

10. Ventilateur selon la revendication 1, **caractérisé en ce qu'**il comprend des moyens d'alimentation électrique (106) fournissant de l'énergie électrique aux moyens de pilotage (40), à la turbine motorisée (41) et/ou au dispositif de commande électromécanique (20).

11. Ventilateur selon l'une des revendications 1 ou 2, **caractérisé en ce qu'**il comprend le dispositif de commande électromécanique (20) comprend un actionneur (21) venant agir contre la face arrière (2.2) du clapet (2) pour le repousser en direction du siège de clapet (3).

12. Ventilateur selon la revendication 11, **caractérisé en ce que** l'actionneur (21) est solidarisé à ladite face arrière (2.2) du clapet (2).

13. Ventilateur selon l'une des revendication 1, 2 ou 11, **caractérisé en ce que** l'actionneur (21) est mû par un électro-aimant proportionnel (VCA).

14. Ventilateur selon l'une des revendications 1, 2 ou 11, **caractérisé en ce que** dispositif de commande électromécanique (20) est au moins partiellement agencé dans un boitier (23) venant se solidariser au corps de la valve (6) de PEP.

15. Ventilateur selon la revendication 14, **caractérisé en ce que** l'actionneur (21) est agencé mobile dans le boitier (23).

Fig. 1

Fig. 2

Fig. 3

Fig. 4

Fig. 5

Fig. 6

# EP 4 785 980 A1

**Europäisches Patentamt**
**European Patent Office**
**Office européen des brevets**

## RAPPORT DE RECHERCHE EUROPEENNE

Numéro de la demande

EP 25 22 2369

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | FR 3 118 584 A1 (AIR LIQUIDE MEDICAL SYSTEMS [FR]) 8 juillet 2022 (2022-07-08) * alinéa [0030] - alinéa [0037]; figures 1-3 * | 1-15 | INV.<br>A61M16/20<br>A61M16/00 |
| A | EP 4 406 575 A1 (AIR LIQUIDE MEDICAL SYSTEMS [FR]) 31 juillet 2024 (2024-07-31) * alinéa [0047]; figure 1 * * alinéa [0047] * | 1-15 | |
| A | US 6 102 038 A (DEVRIES DOUGLAS F [US]) 15 août 2000 (2000-08-15) * figure 2b * | 1-15 | |
| A | FR 3 147 504 A1 (AIR LIQUIDE MEDICAL SYSTEMS [FR]; AIR LIQUIDE MEDICAL SYSTEMS SRL [IT]) 11 octobre 2024 (2024-10-11) * alinéa [0055] - alinéa [0056] * | 1-15 | |
| A | FR 3 122 581 A1 (AIR LIQUIDE MEDICAL SYSTEMS [FR]; AIR LIQUIDE MEDICAL SYSTEMS SRL [IT]) 11 novembre 2022 (2022-11-11) * revendication 7 * | 1-15 | **DOMAINES TECHNIQUES RECHERCHES (IPC)**<br>A61M |
| A | DE 602 09 292 T2 (MAQUET CRITICAL CARE AB [SE]) 2 novembre 2006 (2006-11-02) * figure 1 * | 1-15 | |
| A | EP 2 289 583 A2 (RESMED PARIS [FR]) 2 mars 2011 (2011-03-02) * alinéa [0066]; figures * * alinéa [0066] * | 1-15 | |

Le présent rapport a été établi pour toutes les revendications

| Lieu de la recherche | Date d'achèvement de la recherche | Examinateur |
|---|---|---|
| Munich | 27 avril 2026 | Valfort, Cyril |

EPO FORM 1503 03.82 (P04C02)

EP 4 785 980 A1

## ANNEXE AU RAPPORT DE RECHERCHE EUROPEENNE
## RELATIF A LA DEMANDE DE BREVET EUROPEEN NO.

EP 25 22 2369

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-04-2026

| Document brevet cité au rapport de recherche | | Date de publication | Membre(s) de la famille de brevet(s) | | Date de publication |
|---|---|---|---|---|---|
| FR 3118584 | A1 | 08-07-2022 | AUCUN | | |
| EP 4406575 | A1 | 31-07-2024 | EP | 4406575 A1 | 31-07-2024 |
| | | | ES | 3053421 T3 | 22-01-2026 |
| | | | FR | 3145096 A1 | 26-07-2024 |
| US 6102038 | A | 15-08-2000 | AT | E419824 T1 | 15-01-2009 |
| | | | AT | E517602 T1 | 15-08-2011 |
| | | | BR | 9910456 A | 04-09-2001 |
| | | | CY | 1112380 T1 | 09-12-2015 |
| | | | DK | 2044921 T3 | 29-08-2011 |
| | | | EP | 1077666 A1 | 28-02-2001 |
| | | | EP | 2044921 A1 | 08-04-2009 |
| | | | ES | 2367351 T3 | 02-11-2011 |
| | | | JP | 4087562 B2 | 21-05-2008 |
| | | | JP | 2002515297 A | 28-05-2002 |
| | | | PT | 2044921 E | 27-09-2011 |
| | | | US | 6102038 A | 15-08-2000 |
| | | | WO | 9959517 A1 | 25-11-1999 |
| FR 3147504 | A1 | 11-10-2024 | AUCUN | | |
| FR 3122581 | A1 | 11-11-2022 | CA | 3152045 A1 | 06-11-2022 |
| | | | EP | 4085958 A1 | 09-11-2022 |
| | | | ES | 2972468 T3 | 12-06-2024 |
| | | | FR | 3122581 A1 | 11-11-2022 |
| DE 60209292 | T2 | 02-11-2006 | DE | 60209292 T2 | 02-11-2006 |
| | | | EP | 1336782 A2 | 20-08-2003 |
| | | | JP | 2003240151 A | 27-08-2003 |
| | | | US | 2003151012 A1 | 14-08-2003 |
| EP 2289583 | A2 | 02-03-2011 | CN | 1764486 A | 26-04-2006 |
| | | | EP | 2289583 A2 | 02-03-2011 |
| | | | FR | 2852853 A1 | 01-10-2004 |

EPO FORM P0460

Pour tout renseignement concernant cette annexe : voir Journal Officiel de l'Office européen des brevets, No.12/82

13